# EUROPEAN PATENT SPECIFICATION

(11) **EP 3 310 348 B1**
(45) Date of publication and mention of the grant of the patent: **05.08.2020**
(21) Application number: 16734831.7
(22) Date of filing: 22.06.2016
(51) Int. Cl.: A61K 9/70, A61K 47/12, A61K 47/14, A61K 47/22, A61K 31/445

(54) **TRANSDERMAL ADHESIVE COMPOSITION COMPRISING A POORLY SOLUBLE THERAPEUTIC AGENT**
TRANSDERMALE KLEBSTOFFZUSAMMENSETZUNG MIT EINEM SCHWERLÖSLICHEN THERAPEUTIKUM
COMPOSITION ADHÉSIVE TRANSDERMIQUE COMPRENANT UN AGENT THÉRAPEUTIQUE FAIBLEMENT SOLUBLE

(30) Priority: 22.06.2015 US 201562183108 P
(43) Date of publication of application: 25.04.2018
(73) Proprietor: Corium, Inc., Menlo Park, CA 94025 (US)
(72) Inventor: JAIN, Amit K., Milpitas, California 95035 (US); LEE, Eun Soo, Redwood City, California 94061 (US); SINGH, Parminder, Union City, California 94587 (US)
(74) Representative: D Young & Co LLP
(86) International application number: PCT/US2016/038792
(87) International publication number: WO 2016/209982

(56) References cited:
- EP-A1- 2 016 941
- WO-A1-2012/097197
- US-A1- 2008 038 328
- US-A1- 2014 370 076
- DATABASE WPI Section Ch, Week 201414 Thomson Scientific, London, GB; Class A14, AN 2014-C88308 XP002761690, KATO H: "Patch used for treating Alzheimer-type dementia, comprises support portion, adhesive layer, donepezil and/or its hydrochloride, and additive chosen from isostearic acid, 2-cetyl ethylhexanoate, and hexadecyl isostearate", -& JP 2014 028791 A (SAITAMA DAIICHI SEIYAKU KK) 13 February 2014 (2014-02-13)
- DATABASE WPI Section Ch, Week 201348 Thomson Scientific, London, GB; Class B05, AN 2008-F37689 XP002761691, AIDA K; MICHINAKA Y; TAKEUCHI A; TERAHARA T; UCHIDA N: "Adhesive patch useful in pharmaceuticals, for delivering drugs, provides single surface of support with adhesive layer, where adhesive layer contains drug in solution state and crystalline state", -& JP 5 243254 B2 ((HISM) HISAMITSU PHARM CO LTD) 24 July 2013 (2013-07-24)
- DATABASE WPI Section Ch, Week 201342 Thomson Scientific, London, GB; Class A96, AN 2013-G75464 XP002761692, FANG L; QUAN P; ZHANG Y: "Donepezil percutaneous absorption patch comprises pressure-sensitive adhesive layer, transdermal absorption enhancer and back lining layer which includes polyethylene, polyester and ethylene-vinyl acetate copolymer", -& CN 102 895 217 A ((UYSH-N) UNIV SHENYANG PHARM) 30 January 2013 (2013-01-30)
- JOONHO CHOI ET AL: "Effect of fatty acids on the transdermal delivery of donepezil: In vitro and in vivo evaluation", INTERNATIONAL JOURNAL OF PHARMACEUTICS, ELSEVIER BV, NL, vol. 422, no. 1, 20 October 2011 (2011-10-20), pages 83-90, XP028351842, ISSN: 0378-5173, DOI: 10.1016/J.IJPHARM.2011.10.031 [retrieved on 2011-10-20]

## Description

### CROSS-REFERENCE TO RELATED APPLICATIONS

This field is intentionally left blank.

### TECHNICAL FIELD

The subject matter described herein relates to compositions for the transdermal administration of donepezil that is poorly soluble in an adhesive matrix.

### BACKGROUND

Donepezil is an acetylcholinesterase inhibitor with the chemical structure 2,3-Dihydro-5,6-dimethoxy-2-[[1-(phenylmethyl)-4-piperidinyl]methyl]-1*H*-inden-1-one: 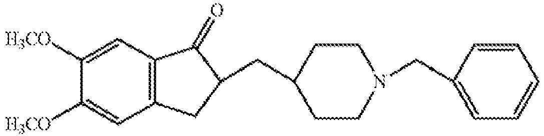 Donepezil has a molecular weight of 379.5 and is lipophilic (Log*P* value 3.08-4311).

U.S. Patent No. 4,895,841 to Eisai Co., Ltd. describes cyclic amine compounds including donepezil for use in treating dementia including Alzheimer senile dementia, Huntington's chorea, Pick's disease, and ataxia.

An oral tablet of donepezil hydrochloride (Aricept®) is approved in the U.S. for use in treating Alzheimer's dementia. Oral donepezil may be associated with adverse cholinergic effects related to the gastrointestinal system including nausea, vomiting and diarrhea as well as sleep disturbance. Further, oral administration of donepezil is associated with frequent plasma fluctuations. Oral donepezil is rapidly absorbed and peak plasma levels (Cₘₐₓ) are reached in about 3 hours (Tiseo et al., Br J Clin Pharmacol, 1998, 46(Suppl 1):13-18). Due to the nature of cognitive disorders, oral medications may be subject to problems with patient compliance especially for formulations that need to be taken throughout the day.

Delivery of medications by transdermal, injection, or rectal (suppositories) routes to patients suffering from cognitive disorders has been investigated. JP 2014 028791 A discloses a patch containing donepezil in which the precipitation of crystals of donepezil is suppressed. U.S. Patent No. 7,858,114 describes a percutaneous absorption preparation of donepezil for use as a plaster for long term delivery of an anti-dementia drug. U.S. Patent No. 2014/0370076 describes a transdermal drug delivery system comprising donepezil or a salt thereof that uses an acrylate-rubber hybrid adhesive that is prepared by a process without n-hexane. Other transdermal delivery systems proposed use an overlay or other rate-limiting membrane to control delivery of the drug from the transdermal device, see e.g. U.S. Published Application No. 2010/0178307 which describes the use of a first and second overlay. Despite these teachings, there are no donepezil transdermal patches or devices available in the United States.

Delivery of anti-dementia drugs over a long period of time (e.g. several days or more) is difficult. Transdermal delivery of basic drugs including donepezil can be especially difficult due to poor skin permeability. Further, some active agents have poor or low solubility in the adhesives and/or other components used in typical transdermal formulations. Further, there is a need for stable, long term administration of anti-dementia agents (e.g. 1-10 days or more) that provides a stable and effective release of the agent over the administration period and has suitable adhesion for the long term administration.

Therefore, there exists a need for transdermal compositions that address at least these shortcomings.

The foregoing examples of the related art and limitations related therewith are intended to be illustrative and not exclusive. Other limitations of the related art will become apparent to those of skill in the art upon a reading of the specification and a study of the drawings.

### BRIEF SUMMARY

The following aspects and embodiments thereof described and illustrated below are meant to be exemplary and illustrative, not limiting in scope. The invention is defined by the claims. Any subject-matter falling outside the scope of the claims is provided for information purposes only.

It is an object of the present invention to provide compositions to effect transdermal delivery of donepezil.

In one aspect of the invention, a composition for preparing a patch for transdermal delivery of donepezil is provided. The composition comprises:
an adhesive matrix comprising
(a) 5-50 wt% donepezil free base;
(b) 5-50 wt% of at least one adhesive polymer;
(c) 5-40 wt% of at least one solubility enhancer that is an ester of a dicarboxylic acid; and
(d) 1-20 wt% of at least one lipophilic permeation enhancer that is selected from a fatty acid, an α-hydroxy acid, a β-hydroxy acid, and a keto carboxylic acid.

In some embodiments, the fatty acid is an unsaturated fatty acid. In some embodiments, the fatty acid is selected from oleic acid, linoleic acid and linolenic acid. In some embodiments, the keto carboxylic acid is levulinic acid. In some embodiments, the α-hydroxy acid is selected from lactic acid and glycolic acid.

In some embodiments, the at least one solubility enhancer is selected from dimethyl succinate and diethyl succinate.

In some embodiments, the composition further comprises at least a second permeation enhancer. In some embodiments, the second permeation enhancer is selected from methyl laurate, propylene glycol monolaurate, glycerol monolaurate, glycerol monooleate, lauryl lactate, laurydone, and myristyl lactate.

In some embodiments, the polymer adhesive is a pressure sensitive polymer adhesive. In some embodiments, the at least one adhesive polymer is selected from an acrylic polymer, an acrylate polymer, an acrylic ester polymer, polymers comprising a pyrrolidone group, polyisobutylenes, polybutenes, polymers comprising an acetate group, derivatives and co-polymers thereof. In some embodiments, the acrylate polymer is present in the adhesive matrix at 35-50 wt%. In some embodiments, the at least one adhesive polymer is selected from a cross-linked polyvinylpyrrolidone, a soluble polyvinylpyrrolidone, and co-polymers thereof. In some embodiments, the at least one adhesive polymer is a polyvinylpyrrolidone-vinyl acetate copolymer present in the adhesive matrix at 5-35 wt%. In some embodiments, the at least one adhesive polymer is a mixture of polyisobutylene and polybutene polymers where the mixture is present in the adhesive matrix at 35-50 wt%.

In some embodiments, the composition further comprises a gelling agent, which may be a cellulose derivative or a carbomer. In some embodiments, the composition further comprises one or more matrix modifiers. In some embodiments, the matrix modifier is selected from the group consisting of fumed silica, colloidal silicon dioxide, a cellulose derivative, polyacrylamide, polyacrylic acid, a polyacrylic acid salt, kaolin, bentonite and combinations thereof.

In a second aspect of the invention, a composition for preparing a patch for transdermal delivery of donepezil is provided. The composition comprises an adhesive matrix comprising: 5-20 wt% donepezil in free base form; 2-6 wt% lauryl lactate or lauryl pyrrolidone carboxylate; 5-20 wt% dimethyl succinate; 0-15% dimethyl sulfoxide; 1-10 wt% levulinic acid or lactic acid; 3-20 wt% fumed silica and/or cross-linked polyvinyl pyrrolidone; and 35 to 80 wt% of an adhesive comprising a mixture of polyisobutylene and polybutene or a hydrogenated polybutene.

Herein it is also disclosed a composition for preparing a patch for transdermal delivery of donepezil. The composition comprises an adhesive matrix comprising: about 5-20 wt% donepezil in free base form; about 2-6 wt% lauryl lactate; about 5-15 wt% of a vinyl acetate and *N*-vinyl pyrrolidone co-polymer; about 40-60 wt% of an acrylic adhesive; and about 5-20 wt% of an adhesive comprising polyisobutylene and polybutene.

In another aspect of the disclosure, a transdermal delivery patch is provided. In some embodiments, the patch comprises a backing layer; an adhesive layer comprising donepezil free base, at least one acid lipophilic permeation enhancer, at least one solubility enhancer, and at least one adhesive polymer; and a release liner. In some embodiments, the release liner is a silicone coated material. In some embodiments, the release liner is a silicone coated PET, fluorocarbon, or fluorocarbon coated PET. In some embodiments, the adhesive layer comprises about 5-50 wt% of the donepezil base. In some embodiments, the adhesive layer is an adhesive matrix.

In some embodiments, the adhesive layer comprises at least about 1-20 wt% of the at least one solubility enhancer. In some embodiments, the at least one solubility enhancer is selected from selected from a dibasic ester, an ester of a dicarboxylic acid, and a dialkylsulfoxide. In some embodiments, the ester of a dicarboxylic acid is selected from dimethyl succinate and diethyl succinate.

In some embodiments, the adhesive layer comprises about 1-20 wt% of the at least one acid lipophilic permeation enhancer. In some embodiments, the at least one acid lipophilic permeation enhancer is selected from a fatty acid, an α-hydroxy acid, a β-hydroxy acid, and a keto carboxylic acid. In some embodiments, the fatty acid is selected from an unsaturated fatty acid and a saturated fatty acid. In some embodiments, the α-hydroxy acid or β-hydroxy acid is selected from glycolic acid, lactic acid, β-hydroxy propionic acid and β-hydroxy butyric acid. In some embodiments, the keto acid is selected from levulinic acid, acetoacetic acid and pyruvic acid.

In some embodiments, the adhesive layer further comprises at least a second permeation enhancer. In some embodiments, the second permeation enhancer is selected from an ester of a dicarboxylic acid or a dialkylsulfoxide. In some embodiments, the ester of dicarboxylic acid is selected from a dialkyl carboxylic acid and dimethyl adipate. In some embodiments, the second permeation enhancer is selected from methyl laurate, propylene glycol monolaurate, glycerol monolaurate, glycerol monooleate, lauryl lactate, laurydone, myristyl lactate and lauryl pyrrolidone carboxylate.

In some embodiments, the adhesive layer further comprises at least one matrix modifier. In some embodiments, the at least one matrix modifier is selected from the group consisting of fumed silica, colloidal silicon dioxide, cross-linked polyvinylpyrrolidone, soluble polyvinylpyrrolidone, a cellulose derivative, polyacrylamide, polyacrylic acid, a polyacrylic acid salt, kaolin, bentonite and combinations thereof.

In some embodiments, the at least one adhesive polymer comprises at least one of a polyacrylate, a polymethacrylate derivative, and copolymers thereof. In some embodiments, the at least one adhesive polymer comprises a mixture of a polyisobutylene and a polybutene polymer.

In a further aspect of the disclosure, a method of making a patch for the transdermal delivery of an active agent is provided. In some embodiments, the active agent is donepezil. In some embodiments, the method comprises: (a) preparing a donepezil solution by dissolving donepezil in a mixture of (i) one or more solubility enhancers, (ii) one or more permeation enhancers, and (iii) a suitable solvent; (b) preparing an adhesive polymer solution by (i) dissolving or dispersing one or more hydrophilic polymers in the donepezil solution of (a) and/or dissolving one or more hydrophobic polymers in a suitable solvent and mixing the hydrophobic polymer solution with the donepezil solution of (a) to form a homogenous or dispersed mixture; (c) coating the adhesive drug formulation on a silicone-coated polyethylene terephthalate film; and (d) drying the coated film. In some embodiments, the at least one hydrophilic polymer is dissolved or dispersed in a suitable solvent or a mixture of solvents prior to dissolving or dispersing in the donepezil solution.

In some embodiments, the at least one hydrophilic polymer is dissolved or dispersed in the solvent at a concentration of about 4 to 35 %w/w. In some embodiments, the at least one hydrophobic polymer is a mixture of polyisobutylene and polybutene polymers. In some embodiments, the hydrophilic polymer is an acrylic ester polymer.

In some embodiments, the adhesive drug formulations are coated to a dry thickness of about 90 mm. In some embodiments, drying comprises drying the film at a suitable temperature and duration to maintain the required excipient content. In some embodiments, drying comprises drying at about 70 °C for about 20 minutes. In some embodiments, the method further comprises applying a backing layer onto the adhesive layer opposite the silicone-coated PET film layer.

Additional embodiments of the present methods and compositions, and the like, will be apparent from the following description, drawings, examples, and claims. As can be appreciated from the foregoing and following description, each and every feature described herein, and each and every combination of two or more of such features, is included within the scope of the present disclosure provided that the features included in such a combination are not mutually inconsistent.

Additional aspects and advantages of the present invention are set forth in the following description and claims, particularly when considered in conjunction with the accompanying examples and drawings.

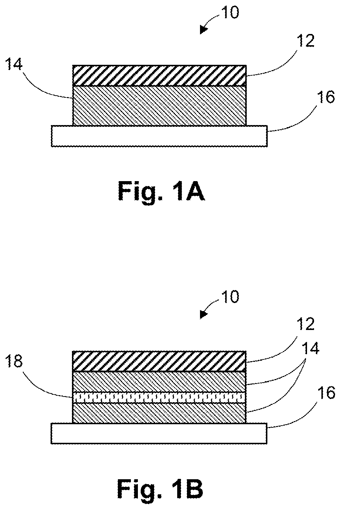

FIGS. 1A-1B are illustrations of transdermal patch configurations in some embodiments.

### DETAILED DESCRIPTION

### I. Definitions

Various aspects now will be described more fully hereinafter. Such aspects may, however, be embodied in many different forms and should not be construed as limited to the embodiments set forth herein; rather, these embodiments are provided so that this disclosure will be thorough and complete, and will fully convey its scope to those skilled in the art.

The present compositions, devices, and methods are not limited to the specific polymers, excipients, cross-linking agents, additives, manufacturing processes, or adhesive products described herein. It will be understood that the particular terminology used herein is for the purpose of describing particular embodiments and is not intended to be limiting.

Where a range of values is provided, it is intended that each intervening value between the upper and lower limit of that range and any other stated or intervening value in that stated range is encompassed within the disclosure. For example, if a range of 1 µm to 8 µm is stated, it is intended that 2 µm, 3 µm, 4 µm, 5 µm, 6 µm, and 7 µm are also explicitly disclosed, as well as the range of values greater than or equal to 1 µm and the range of values less than or equal to 8 µm.

The singular forms "a," "an," and "the" include plural referents unless the context clearly dictates otherwise. Thus, for example, reference to a "polymer" includes a single polymer as well as two or more of the same or different polymers, reference to an "excipient" includes a single excipient as well as two or more of the same or different excipients, and the like.

The use of terms of order or importance, including "first" and "second", is to distinguish and identify individual elements and does not denote or imply a particular order or importance unless clearly indicated by context.

The term "active agent" as used herein refers to a chemical material or compound suitable for topical or transdermal administration and that induces a desired effect. The terms include agents that are therapeutically effective, prophylactically effective, and cosmetically effective agents. The terms "active agent", "drug" and "therapeutic agent" are used interchangeably herein.

An "adhesive matrix" as described herein includes matrices made in one piece, for example, matrices made via solvent casting or extrusion as well as matrices formed in two or more portions that are then pressed or joined together.

The term "skin" as used herein refers to skin or mucosal tissue, including the interior surface of body cavities that have a mucosal lining. The term "skin" should be interpreted as including "mucosal tissue" and vice versa.

"Donepezil" as used herein refers to 2,3-dihydro-5,6-dimethoxy-2-[[1-(phenylmethyl)-4-piperidinyl]methyl]-1*H*-inden-1-one.

The term "therapeutically effective amount" as used herein refers to the amount of an active agent that is nontoxic but sufficient to provide the desired therapeutic effect. The amount that is "effective" will vary from subject to subject, depending on the age and general condition of the individual, the particular active agent or agents, and the like as known to those skilled in the art.

The terms "transdermal" or "transdermal delivery" as used herein refer to administration of an active agent to a body surface of an individual so that the agent passes through the body surface, e.g., skin, and into the individual's blood stream. The term "transdermal" is intended to include transmucosal administration, i.e., administration of a drug to the mucosal (e.g., sublingual, buccal, vaginal, rectal) surface of an individual so that the agent passes through the mucosal tissue and into the individual's blood stream.

### II. Compositions / Devices

The compositions and devices described herein are designed for transdermal administration of an active agent such as donepezil. The compositions may be used in devices, patches or systems suitable for transdermal delivery of the agent. The active agent is discussed with reference to donepezil below. However, it will be appreciated that the discussion is applicable to other suitable active agents.

One impediment to transdermal delivery is that many active agents such as donepezil have low solubility in the adhesive matrix. In embodiments, the present compositions are suitable for active agents having low solubility, that is, a solubility of less than about 3 wt% in a rubber or silicone adhesive. In some embodiments, the active agent has a solubility of least about 10 wt% in the composition. The composition comprises an adhesive or adhesive matrix that comprises the active agent, at least one lipophilic penetration enhancer, and at least one solubilizing agent to increase solubility of the agent in the matrix and/or prevent precipitation of the agent in the matrix. In the present invention, the active agent is donepezil in free base form. In some embodiments, the donepezil free base is in non-crystalline form. The composition comprises an adhesive matrix comprising the active agent, one or more adhesive polymers, a dibasic ester, and an acid. The adhesive matrix comprises 5-50 wt% of the active agent. In some embodiments, the adhesive matrix comprises 5-40 wt%, 5-30 wt%, 5-25 wt%, 5-20 wt%, 5-15 wt%, 5-10 wt%, 10-50 wt%, 10-40 wt%, 10-30 wt%, 10-25 wt%, 10-20 wt%, 10-15 wt%, 20-50 wt%, 20-40 wt%, 20-30 wt%, 20-25 wt%, 30-50 wt%, 30-40 wt%, or 40-50 wt% of the active agent.

The adhesive matrix comprises one or more polymers. One or more of the polymers is an adhesive polymer. In embodiments, the adhesive matrix comprises at least about 25-80 wt% of polymers relative to the weight of the adhesive matrix (inclusive of sub-ranges). In embodiments, the matrix comprises at least about 35-80%, 30-75%, at least about 40-75%, at least about 50-75%, at least about 60-75%, at least about 25-70%, at least about 30-70%, at least about 40-70%, at least about 50-70%, at least about 60-70%, at least about 25-60%, at least about 30-60%, at least about 40-60%, at least about 50-60%, at least about 25-50%, at least about 30-50%, at least about 40-50%, at least about 25-40%, at least about 30-40%, or at least about 25-30% of the polymers (all percentages in wt%). It will be appreciated that the adhesive matrix may include one or more or at least one polymers. In embodiments, the adhesive matrix comprises at least about 5-75% of an individual polymer relative to the total weight of the polymers in the matrix. In embodiments, the adhesive matrix comprises at least about 5-10%, 5-15%, 5-20%, 5-25%, 5-30%, 5-40%, 5-50%, 5-60%, 5-70%, 5-75%, 10-15%, 10-20%, 10-20%, 10-25%, 10-30%, 10-40%, 10-50%, 10-60%, 10-70%, 10-75%, 15-20%, 15-25%, 15-30%, 15-40%, 15-50%, 15-60%, 15-70%, 15-75%, 20-25%, 20-30%, 20-40%, 20-50%, 20-60%, 20-70%, 20-75%, 25-30%, 25-40%, 25-50%, 25-60%, 25-70%, 25-75%, 30-40%, 30-50%, 30-60%, 30-70%, 30-75%, 40-50%, 40-60%, 40-70%, 40-75%, 50-60%, 50-70%, 50-75%, 60-70%, 60-75%, or 70-75% of an individual polymer. The adhesive matrix according to the first aspect of the invention comprises 5-50 wt% of at least one adhesive polymer.

In one embodiment, the matrix is comprised of one or more pressure sensitive adhesive polymers. In embodiments, the adhesive polymer is an acrylic polymer. In some embodiments, the adhesive polymer is an acrylic pressure sensitive adhesive polymer. In embodiments, the acrylic polymer is a polyacrylate adhesive polymer. An acrylic pressure sensitive adhesive polymer is a polyacrylate that is a polymer of copolymer of a monomer or monomers selected from acrylic acid esters and methacrylic acid esters. Other monomers, such as acrylic acid and vinyl acetate, may be present. In embodiments, the acrylic polymer is based on acrylic esters such as 2-ethylhexyl acrylate (2-EHA) and ethyl acrylate. In some embodiments, the polyacrylate polymer is a polymer or a copolymer of a monomer or monomers selected from acrylic acid and vinyl acetate. In embodiments, the acrylic polymer adhesive has pendent carboxyl (-COOH) or hydroxyl (-OH) functional groups. In embodiments, the acrylic polymer adhesive comprises at least one of polyacrylate, polymethacrylate, derivatives thereof, and co-polymers thereof. In embodiments, the acrylic adhesive is comprised of an acrylate copolymer comprising acrylic ester monomers or vinyl acetate monomers. Exemplary acrylate copolymers are sold under the trade-name DURO-TAK and include, but are not limited to, DURO-TAK 387-2516, 387-2051, and 387-2074.

In some embodiments, the matrix is comprised of one or more polymers comprising a pyrrolidone group, polyisobutylenes, polybutenes, mixtures and co-polymers thereof. In embodiments, the adhesive matrix comprises a blend or mixture of polyisobutylene and polybutene polymers. Polyisobutylene is a vinyl polymer comprised of the isobutylene monomer. Polybutene is a viscous, non-drying, liquid polymer, prepared by copolymerization of 1- and 2-butene with a small quantity of isobutylene. In some embodiments, the polybutene in one embodiment has a molecular weight of between about 750-6000 Daltons, preferably between about 900-4000 Daltons, and preferably between about 900-3000 Daltons. In some embodiments the mixture comprises polybutene in the polyisobutylene blend at about 40 weight percent. More generally, the polybutene is present in the polyisobutylene blend in an amount between 20-50 weight percent, or between 25-45 weight percent. In some embodiments, the adhesive matrix does not include an acrylate-rubber adhesive.

In embodiments, the copolymer is selected from a polyvinylpyrrolidone/vinyl acetate copolymer, an acrylic acid/vinyl acetate copolymer, and a vinyl acetate/ethylene acetate copolymer. In one embodiment, the copolymer is a vinyl acetate/*N*-vinylpyrrolidone copolymer such as the copolymer sold as Plasdone™ S630 (Ashland). In another embodiment, the polyvinylpyrrolidone-vinyl acetate copolymer is a linear random copolymer of n-vinyl-2-pyrrolidone and vinyl acetate. In one embodiment, the copolymer is a 60:40 copolymer of n-vinyl-2-pyrrolidone and vinyl acetate. In exemplary embodiments, the acrylic polymer is a pressure sensitive adhesive such as the polymers and copolymers sold as DURO-TAK™. In specific, but not limiting embodiments, the matrix comprises at least one acrylate co-polymer selected from DURO-TAK™ nos. 387-2516, 387-2051 and 387-2074. In embodiments, the adhesive matrix comprises polyvinylpyrrolidone (PVP). PVP is a water-soluble polymer comprised of the *N*-vinylpyrrolidone monomer.

In some embodiments, the adhesive matrix comprises hydrophilic and hydrophobic polymers. One skilled in the art may determine suitable hydrophilic and/or hydrophobic polymers by means known in the art. By way of example, a polyvinylpyrrolidone-vinyl acetate copolymer is a hydrophilic polymer and each of polyisobutylene, polyisobutylene and polybutene mixture, and an acrylic acid/vinyl acetate copolymer are hydrophobic. In embodiments, the polymers may include a cross-linker.

In embodiments, the adhesive matrix comprises at least one lipophilic permeation enhancer. In some embodiments, the permeation enhancer is an acid. In embodiments, the permeation enhancer is selected from fatty acids, fatty alcohol esters, α-hydroxy acids, β-propionic acids, dibasic esters, and esters of dicarboxylic acids. It will be appreciated that the adhesive matrix may include at least one permeation enhancer. In some embodiments, the adhesive matrix comprises at least two permeation enhancers.

In some embodiments, the fatty acid is a C₅-C₂₀ fatty acid. In some embodiments, the fatty acid is a C₅-C₈ or a C₈-C₂₀ fatty acid. The fatty acid may be a saturated or an unsaturated fatty acid. In some embodiments, the fatty acid is selected from one or more of valeric acid, caprylic acid, capric acid, lauric acid, myristic acid, palmitic acid, stearic acid, oleic acid, linoleic acid, linolenic acid, levulinic acid, and arachidic acid.

In some embodiments, the fatty alcohol ester has the chemical formula CH₃(CH₂)ₘCOOR' or CH₃(CH₂)ₘ-OCOCHR₁R₂ where m is an integer between 8 and 14, R' is a lower C₁-C₃ alkyl residue that is unsubstituted or substituted with 1, 2 or 3 hydroxyl groups, and R₁ and R₂ are individually hydrogen, hydroxyl, or a lower C₁-C₂ alkyl. In some embodiments, one of R₁ and R₂ is hydrogen and the other is hydroxyl. In other embodiments, one of R₁ and R₂ is hydroxyl and the other is a lower C₁-C₂ alkyl.

In some embodiments, the α-hydroxy acid is selected from lactic acid and glycolic acid. It will be appreciated that the fatty acid, α-hydroxy acid, and/or β-propionic acids may be any suitable acid that is in liquid form at room temperature and is safe for transdermal administration as known in the art.

In the present invention, the lipophilic permeation enhancer is selected from a fatty acid, an α-hydroxy acid, a β-hydroxy acid and a keto carboxylic acid.

The lipophilic penetration enhancer is included in the adhesive matrix in an amount to provide sufficient transport of at least the active agent across the skin. The amount of lipophilic penetration enhancer, as well as additional penetration or permeation enhancers, may be determined based on the permeability of the active agent. In embodiments, the lipophilic penetration enhancer is included in an amount about between about 1-15% relative to the weight of the adhesive matrix (inclusive of sub-ranges). In embodiments, the lipophilic penetration enhancer is included in an amount of between about 1-5%, 1-10%, 2-5%, 2-10%, 2-15%, 3-5%, 3-10%, 3-15%, 4-5%, 4-10%, 4-15%, 5-10%, 5-15%, 10-15%, etc. relative to the weight of the adhesive matrix (inclusive of sub-ranges). The adhesive matrix according to the first aspect of the invention comprises 1-20 wt% of at least one lipophilic permeation enhancer as claimed.

The at least one solubilizing agent or solubility enhancing agent may be used to increase solubility of the active agent in the adhesive matrix and/or to prevent precipitation of the agent in the matrix. In embodiments, the at least one solubilizing agent or solubility enhancing agent is a dibasic ester. In the present invention, the solubility enhancer is an ester of a dicarboxylic acid. In some embodiments, the dibasic ester is an ester of succinic acid. In some embodiments, the ester of succinic acid is one of dimethyl succinate or diethyl succinate. In embodiments, the solubilizing agent or solubility enhancing agent is included in an amount between about 5-50%, about 5-40%, 5-30%, 5-25%, 5-20%, 5-15%, 5-10%, 10-50%, 10-40%, 10-30%, 10-25%, 10-20%, 10-15%, 15-50%, 15-40%, 15-30%, 15-25%, 15-20%, 20-50%, 20-40%, 20-30%, 20-25%, 25-50%, 25-40%, 25-30%, 30-50%, 30-40%, or 40-50% relative to the weight of the adhesive matrix (inclusive of sub-ranges). The adhesive matrix according to the first aspect of the invention comprises 5-40 wt% of at least one solubility enhancer as claimed.

In some embodiments, the composition includes one or more additives or excipients including, but not limited to, additional penetration or permeation enhancers and/or matrix modifiers.

In some embodiments, a penetration or permeation enhancer (in addition to the lipophilic penetration or permeation enhancer described above) is included in the adhesive matrix. In embodiments, the additional penetration or permeation enhancer is a pyrrolidone compound comprising a 5-membered lactam. In some embodiments, the pyrrolidone compound is selected from one or more of laurydone, a lauric acid ester of pyrrolidone, and pyrrolidone derivatives having a fatty acid chain of at least 5-8 carbons or a fatty acid chain that is longer than 8 carbons.

The penetration or permeation enhancer may be chosen from a wide range of such compounds known in the art. In some embodiments, permeation enhancers for use in the adhesive matrix include, but are not limited to, methyl laurate, propylene glycol monolaurate, glycerol monolaurate, glycerol monooleate, lauryl lactate, myristyl lactate, and dodecyl acetate. Additional permeation enhancers are described in U.S. Patent No. 8,874,879. It will be appreciated that the compositions herein may include one or more or at least one permeation enhancer. In embodiments, the penetrating or permeating enhancer is included in an amount between about 1-10%, about 2-5%, about 2-10% relative to the weight of the adhesive matrix (inclusive of sub-ranges).

The permeation enhancers may be used to adjust the rate of delivery and/or dosage administered. The dosage may be affected by the rate of permeation rate from the patch into the blood stream and/or the size (e.g. cm²) of the transdermal device.

The adhesive matrix may further include one or more matrix modifiers. Without wishing to be bound by theory, it is believed that the matrix modifier facilitates homogenization of the adhesive matrix. Sorption of hydrophilic moieties is a possible mechanism for this process. Thus, known matrix modifiers which are to some degree water-sorbent may be used. For example, possible matrix modifiers include colloidal silicone dioxide, fumed silica, cross-linked polyvinylpyrrolidone (PVP), soluble PVP, cellulose derivatives (e.g. hydroxypropyl cellulose (HPC), hydroxyethylcellulose (HEC)), polyacrylamide, polyacrylic acid, a polyacrylic acid salt, or a clay such as kaolin or bentonite. An exemplary commercial fumed silica product is Cab-O-SiI (Cabot Corporation, Boston, Mass.). The hydrophilic mixtures described in U.S. Published Patent Application No. 2003/0170308 may also be employed, for example mixtures of PVP and PEG or of PVP, PEG, and a water-swellable polymer such as Eudragit L100-55. In embodiments, the matrix modifier is individually included in an amount between about 1-25%, about 2-25%, about 5-25%, about 5-7%, about 7-20%, or about 7-25% relative to the weight of the adhesive matrix (inclusive of sub-ranges). In some embodiments, the matrix modifier does not include ethylcellulose.

The composition may also include other conventional additives such as adhesive agents, antioxidants, crosslinking or curing agents, pH regulators, pigments, dyes, refractive particles, conductive species, antimicrobial agents, opacifiers, gelling agents, viscosity modifiers or thickening agents, stabilizing agents, and the like as known in the art. In those embodiments wherein adhesion needs to be reduced or eliminated, conventional detackifying agents may also be used. Other agents may also be added, such as antimicrobial agents, to prevent spoilage upon storage, i.e., to inhibit growth of microbes such as yeasts and molds. Suitable antimicrobial agents are typically selected from the group consisting of the methyl and propyl esters of p-hydroxybenzoic acid (i.e., methyl and propyl paraben), sodium benzoate, sorbic acid, imidurea, and combinations thereof. These additives, and amounts thereof, are selected in such a way that they do not significantly interfere with the desired chemical and physical properties of the adhesive and/or active agent.

The compositions may also contain irritation-mitigating additives to minimize or eliminate the possibility of skin irritation and/or skin damage resulting from the drug, the enhancer, or other components of the composition. Suitable irritation-mitigating additives include, for example: α-tocopherol; monoamine oxidase inhibitors, particularly phenyl alcohols such as 2-phenyl-1-ethanol; glycerin; salicylic acids and salicylates; ascorbic acids and ascorbates; ionophores such as monensin; amphiphilic amines; ammonium chloride; N-acetylcysteine; cis-urocanic acid; capsaicin; and chloroquine. Corticosteriods are also known in art as irritation-mitigating additives.

Methods for preparing or manufacturing the adhesive matrix are also provided. With reference to Example 1, in embodiments, the methods comprise (i) solubilizing one or more polymers in 10-30% wt% of a suitable solvent or solvents, (ii) mixing (i) with at least one solubility enhancer and at least one lipophilic permeation enhancer, (iii) dissolving at least 5 % w/w of an active agent such as donepezil free base to (ii), and forming an adhesive matrix from the adhesive solution that comprises 5-50 wt% of at least one adhesive polymer, 5-40 wt% of a solubility enhancer, and 1-20 wt% of a lipophilic penetration enhancer.

The adhesive matrix may be used as an adhesive layer in a transdermal device, apparatus, or patch. Some exemplary devices are shown in Figures 1A-1B. As seen in Fig. 1A, one exemplary patch 10 includes a backing layer 12, an adhesive matrix layer 14 and an optional release liner 16.

In some embodiments, the backing layer provides a structural element for holding or supporting the adhesive layer. The backing layer may be formed of any suitable material as known in the art. In some embodiments, the backing layer is occlusive. In some embodiments, the backing is preferably impermeable or substantially impermeable to moisture. In one exemplary embodiment, the barrier layer has an MVTR (moisture vapor transmission rate) of less than about 50 g/m²-day. In some embodiments, the backing layer is preferably inert and/or does not absorb components of the adhesive layer, including the active agent. In some embodiments, the backing layer preferably prevents release of components of the adhesive layer through the backing layer. The backing layer may be flexible or nonflexible. The backing layer is preferably at least partially flexible such that the backing layer is able to conform at least partially to the shape of the skin where the patch is applied. In some embodiments, the backing layer is flexible such that the backing layer conforms to the shape of the skin where the patch is applied. In some embodiments, the backing layer is sufficiently flexible to maintain contact at the application site with movement, e.g. skin movement. Typically, the material used for the backing layer should permit the device to follow the contours of the skin or other application site and be worn comfortably on areas of skin such as at joints or other points of flexure, that are normally subjected to mechanical strain with little or no likelihood of the device disengaging from the skin due to differences in the flexibility or resiliency of the skin and the device.

In some embodiments, the backing layer is formed of one or more of a film, non-woven fabric, woven fabric, laminate, and combinations thereof. In some embodiments, the film is a polymer film comprised of one or more polymers. Suitable polymers are known in the art and include elastomers, polyesters, polyethylene, polypropylene, polyurethanes and polyether amides. In some embodiments, the backing layer is formed of one or more of polyethylene terephthalate, various nylons, polypropylene, metalized polyester films, polyvinylidene chloride, and aluminum foil. In some embodiments, the backing layer is a fabric formed of one or more of polyesters such as polyethylene terephthalate, polyurethane, polyvinyl acetate, polyvinylidene chloride and polyethylene. In one particular, but non-limiting embodiment, the backing layer is formed of a polyester film laminate. One particular polyester film laminate is the polyethylene and polyester laminate such as the laminate sold under the name Scotchpak™ #9723.

The device includes at least one adhesive layer adjacent the backing layer. In embodiments, the adhesive layer is an adhesive matrix comprising the active agent as described above. The adhesive layer adheres to the backing layer and/or skin at the administration site. The adhesive layer matrix also serves to release the active agent to the skin.

In embodiments, the device includes a release liner at least partially in contact at least with the adhesive layer to protect the adhesive layer prior to application. The release liner is typically a disposable layer that is removed prior to application of the device to the treatment site. In some embodiments, the release liner preferably does not absorb components of the adhesive layer, including the active agent. In some embodiments, the release liner is preferably impermeable to components of the adhesive layer (including the active agent) and prevents release of components of the adhesive layer through the release liner. In some embodiments, the release liner is formed of one or more of a film, non-woven fabric, woven fabric, laminate, and combinations thereof. In some embodiments, the release liner is a silicone-coated polymer film or paper. In some non-limiting embodiments, the release liner is a silicone-coated polyethylene terephthalate (PET) film, a fluorocarbon film, or a fluorocarbon coated PET film.

In some embodiments, the device further includes a fabric or tie layer 18 within the adhesive matrix. The tie layer may be formed of any suitable material including, but not limited to, polyesters, vinyl acetate polymers and copolymers, polyethylenes, and combinations thereof. In one embodiment, the tie layer is a nonwoven layer of polyester fibers such as the film sold under the name Reemay® (Kavon Filter Products Co.). In embodiments, the tie layer does not affect the rate of release of the active agent from the adhesive layers.

The thickness and/or size of the device and/or adhesive matrix may be determined by one skilled in the art based at least on considerations of wearability and/or required dose. It will be appreciated that the administration site for the device will affect the wearability considerations due to the available size of the administration site and the use of the administration site (e.g. need for flexibility to support movement). In some embodiments, the device and/or adhesive matrix has a thickness of between about 25-500 µm. In some embodiments, the device and/or adhesive matrix has a thickness of between about 50-500 µm. In some embodiments, the patch has a size in the range of about 16 cm²-225 cm². It will be appreciated that the thickness and size provided here are merely exemplary and the actual thickness and or size may be thinner/smaller or thicker/larger as needed for a specific formulation.

An adhesive formulation is cast or otherwise applied to a suitable film such as a release liner and dried to remove all solvents and/or volatile compounds. In some embodiments, the formulation is dried at a temperature between about 5-100 °C. The adhesive matrix is then laminated to a suitable film such as a backing layer or film.

Adhesive formulations and adhesive matrices were prepared to illustrate the embodiments described herein. Examples 1 and 2-7 set forth exemplary formulations and the resulting adhesive matrices using donepezil in free base form.

In Example 1, an adhesive formulation comprising the active agent at 10.0 wt%, an adhesive polymer at 40.0 wt%, a solubility enhancer at 10-30 wt%; and an acid lipophilic permeation enhancer at 3.0 wt%. The adhesive polymer was an acrylic acid/vinyl acetate copolymer. The adhesive matrix further included matrix modifiers and further skin penetration enhancers. The ability to use an active agent such as donepezil in its free base form allows for a lower drug load in the adhesive matrix with similar delivery as the salt forms of the drug. The formulation of Example 1 comprises an ester of a dicarboxylic acid (dimethyl succinate) which is effective to prevent the active agent from precipitating in the polymers. The solubility enhancer also provides a matrix for the drug to dissolve and remain in solution. The increased solubility of the active agent in the adhesive matrix provides greater stability and a resulting increase in shelf life. The formulation of Example 1 also includes an acid permeation enhancer (levulinic acid). The permeation enhancer forms a complex with the active agent thereby increasing the solubility of the active agent in the skin resulting in enhanced permeation of the active agent through the skin.

In Example 3, an adhesive formulation comprising the active agent at 10.0 wt%, adhesive polymers at 45.0-55 wt%, a solubility enhancer at 10-30 wt%; and an acid lipophilic permeation enhancer at 3.0 wt%. The adhesive polymers were comprised of a homogeneous blend of an acrylic acid/vinyl acetate copolymer and a polyvinylpyrrolidone/vinyl acetate copolymer. The adhesive matrix further included matrix modifiers and further skin penetration enhancers. In this example, only the compositions falling within the scope of claim 1 are according to the invention.

In Example 4, an adhesive formulation comprising the active agent at 10.0 wt%, adhesive polymers at 50.0-60.0 wt%, a solubility enhancer at 10-30 wt%; and an acid lipophilic permeation enhancer at 3.0 wt%. The adhesive polymers were comprised of a homogeneous blend of an acrylic acid/vinyl acetate copolymer, a polyvinylpyrrolidone/vinyl acetate copolymer, and an acrylic acid copolymer. The adhesive matrix further included matrix modifiers and further skin penetration enhancers. In this example, only the compositions falling within the scope of claim 1 are according to the invention.

In Example 5, an adhesive formulation comprising the active agent at 10.0 wt% and adhesive polymers at about 56.0 wt%. The adhesive polymers were comprised of a polyisobutylene/polybutene mixture. This adhesive formulation did not include a solubility enhancer or an acid lipophilic permeation enhancer, and is not according to the invention.

In Example 6, an adhesive formulation comprising the active agent at 10.0 wt%, an adhesive polymer at about 56 wt%, a solubility enhancer at 10-30 wt%; and an acid lipophilic permeation enhancer at 3.0 wt%. The adhesive polymer was comprised of a polyisobutylene/polybutene mixture. The adhesive matrix further included a matrix modifier and further skin penetration enhancers. In this example, only the compositions falling within the scope of claim 15 are according to the invention.

In Example 7, an adhesive formulation comprising the active agent at 10.0 wt%, adhesive polymers at about 56 wt%, a solubility enhancer at 10-30 wt%; and an acid lipophilic permeation enhancer at 3.0 wt%. The adhesive polymer was comprised of a polyisobutylene/polybutene mixture. The adhesive matrix further included a matrix modifier and further skin penetration enhancers. In this example, only the compositions falling within the scope of claim 15 are according to the invention.

### III. Methods of Treatment

Based on the exemplary compositions and devices described herein, and the data showing release of donepezil from the adhesive, skin-contacting layer, a method for treating a suitable condition with donepezil is provided.

In embodiments, compositions and devices comprising donepezil are useful for treating, delaying progression, delaying onset, slowing progression, preventing, providing remission, and improvement in symptoms of cognitive disorders or disease are provided herein. In embodiments, compositions and devices comprising donepezil are provided for maintaining mental function including, but not limited to a least one of maintaining thinking, memory, speaking skills as well as managing or moderating one or more behavioral symptoms of a cognitive disorder or disease. In embodiments, the cognitive disorder is Alzheimer's disease. In particular embodiments, the cognitive disorder is Alzheimer's type dementia. In embodiments, compositions and devices comprising donepezil are provided for use in treating, etc. mild, moderate, or severe Alzheimer's disease.

Alzheimer's disease is the most common cause of senile dementia and is characterized by cognitive deficits related to degeneration of cholinergic neurons. Alzheimer's affects 6-8% of people over the age of 65 and nearly 30% of people over the age of 85 (Sozio et al., Neurophsychiatric Disease and Treatment, 2012, 8:361-368), involving the loss of cognitive functioning and behavioral abilities. The causes of Alzheimer's disease are not yet fully understood. As Alzheimer's disease is associated with reduced levels of several cerebral neurotransmitters including acetylcholine (Ach), current treatment includes administering cholinesterase inhibitors. Cholinesterase inhibitors reduce the hydrolysis of acetylcholine in the synaptic cleft by inhibiting cholinesterase and/or butyrylcholinesterase, which increases acetylcholine levels resulting in improved neurotransmission (Sozio *et al.*).

The transdermal devices described herein may be designed for long term use and/or continuous administration of the active agent. The FDA has approved daily oral doses of donepezil of 5 mg, 10 mg, and 23 mg. It will be appreciated that the total dose of the active agent per transdermal device will be determined by the size of the device and the loading of the active agent within the adhesive matrix. In the present invention, the active agent is donepezil in free base form. Lower drug loading of donepezil base may be effective as compared to the salt form (e.g. donepezil hydrochloride). The ability to include lower drug loading to achieve efficacy results in a lower profile for the device (thinner) and/or smaller size, both of which are desirable to reduce discomfort. In some embodiments, the application period for the transdermal device is between about 1-10 days, 1-7 days, 1-5 days, 1-2 days, 3-10 days, 3-7 days, 3-5 days, 5-10 days, and 5-7 days inclusive. In some embodiments, the active agent is released from the adhesive matrix as a continuous and/or sustained release over the application period.

### IV. Examples

The following examples are illustrative in nature and are in no way intended to be limiting.

### EXAMPLE 1

### Preparation of Adhesive Formulation Comprising Donepezil

An acrylate adhesive solution was prepared by dissolving an acrylic acid/vinyl acetate copolymer (DuroTak 387-2516) in a solvent to yield a solution.

An adhesive formulation was prepared by mixing the acrylate adhesive solution, levulinic acid, dimethyl sulfoxide, lauryl lactate, crosslinked polyvinylpyrrolidone, fumed silica (Cab-o-sil) until a homogeneous solution was formed. Donepezil in free base form was added to the solution and vortexed until dissolved. The adhesive formulation had a final composition as follows:

**Adhesive Formulation No. 1**

| | | |
|---|---|---|
| Active Agent | donepezil base | 10.0 wt% |
| Adhesive Polymer(s) | acrylic acid/vinyl acetate copolymer | 40.0 wt% |
| Solubility Enhancer | dimethyl succinate | 10-30 wt% |
| Lipophilic Permeation Enhancer | levulinic acid | 3.0 wt% |
| Matrix Modifier | crosslinked polyvinylpyrrolidone | 15.0 wt% |
| | fumed silica | 7.0 wt% |
| Skin Penetration Enhancer | lauryl lactate | 2-4 wt% |
| | dimethyl sulfoxide | 0-5 wt% |

### EXAMPLE 2

### Preparation of Transdermal Device Comprising Donepezil

An adhesive matrix was prepared by coating the adhesive formulation of Example 1 onto a silicon-coated polyethylene terephthalate release liner at a wet thickness of 0.508 mm (20 mils) and then drying at about 70 °C for about 20 minutes. After drying, the adhesive drug formulation has a dry thickness of about 90 mm.

A backing layer (Scotchpak 9732) was laminated onto the adhesive matrix and transdermal devices of 10 cm² were die cut from the laminate.

### EXAMPLE 3

Preparation of Adhesive Formulation Comprising Donepezil. In this example, only the compositions falling within the scope of claim 1 are according to the invention

An adhesive formulation was prepared substantially as described in Example 1 to yield an adhesive formulation with the following composition:

**Adhesive Formulation No. 2**

| | | |
|---|---|---|
| Active Agent | donepezil base | 10 wt% |
| Adhesive Polymer(s) | polyvinylpyrrolidone/vinyl acetate copolymer | 5-15 wt% |
| | acrylic acid/vinyl acetate copolymer | 40.0 wt% |
| Solubility Enhancer | dimethyl succinate | 10-30 wt% |
| Lipophilic Permeation Enhancer | levulinic acid | 3.0 wt% |
| Matrix Modifier | crosslinked polyvinylpyrrolidone | 15.0 wt% |
| | fumed silica | 7.0 wt% |
| Skin Penetration Enhancer | lauryl lactate | 2-4 wt% |
| | dimethyl sulfoxide | 0-5 wt% |

### EXAMPLE 4

Preparation of Adhesive Formulation Comprising Donepezil. In this example, only the compositions falling within the scope of claim 1 are according to the invention

An adhesive formulation was prepared substantially as described in Example 1 to yield an adhesive formulation with the following composition:

**Adhesive Formulation No. 3**

| | | |
|---|---|---|
| Active Agent | donepezil base | 10.0 wt% |
| Adhesive Polymer(s) | polyvinylpyrrolidone vinyl acetate copolymer | 5-15 wt% |
| | acrylic acid/vinyl acetate copolymer | 40.0 wt% |
| | acrylic acid copolymer | 5.0 wt% |
| Solubility Enhancer | dimethyl succinate | 10-30 wt% |
| Lipophilic Permeation Enhancer | levulinic acid | 3 wt% |
| Matrix Modifier | crosslinked polyvinylpyrrolidone | 15.0 wt% |
| | fumed silica | 7.0 wt% |
| Skin Penetration Enhancer | lauryl lactate | 2-4 wt% |
| | dimethyl sulfoxide | 0-5 wt% |

### EXAMPLE 5

Preparation of Adhesive Formulation Comprising Donepezil (not according to the invention)

An adhesive formulation was prepared substantially as described in Example 1 to yield an adhesive formulation with the following composition:

**Adhesive Formulation No. 4**

| | | |
|---|---|---|
| Active Agent | donepezil base | 20.0 wt% |
| Adhesive Polymer(s) | polyvinylpyrrolidone vinyl acetate copolymer | 10-15 wt% |
| | acrylic acid/vinyl acetate copolymer | 40-60 wt% |
| | polyisobutylene/polybutene mixture | 5-20 wt% |
| Solubility Enhancer | | 0 |
| Lipophilic Permeation Enhancer | | 0 |
| Skin Penetration Enhancer | lauryl lactate | 2-6 wt% |

### EXAMPLE 6

Preparation of Adhesive Formulation Comprising Donepezil. In this example, only the compositions falling within the scope of claim 15 are according to the invention

An adhesive formulation was prepared substantially as described in Example 1 to yield an adhesive formulation with the following composition:

**Adhesive Formulation No. 5**

| | | |
|---|---|---|
| Active Agent | donepezil base | 10.0 wt% |
| Adhesive Polymer(s) | polyisobutylene/polybutene mixture | ∼56 wt% |
| Solubility Enhancer | dimethyl succinate | 10-30 wt% |
| Lipophilic Permeation Enhancer | levulinic acid | 3.0 wt% |
| Matrix Modifier | fumed silica | 7.0 wt% |
| Skin Penetration Enhancer | dimethyl sulfoxide | 0-5 wt% |
| | lauryl lactate | 2-4 wt% |

### EXAMPLE 7

Preparation of Adhesive Formulation Comprising Donepezil. In this example, only the compositions falling within the scope of claim 15 are according to the invention

An adhesive formulation was prepared substantially as described in Example 1 to yield an adhesive formulation with the following composition:

**Adhesive Formulation No. 6**

| | | |
|---|---|---|
| Active Agent | donepezil base | 10.0 wt% |
| Adhesive Polymer(s) | polyisobutylene/polybutene mixture | ∼56 wt% |
| Solubility Enhancer | dimethyl succinate | 10-30 wt% |
| Lipophilic Permeation Enhancer | levulinic acid | 3.0 wt% |
| Matrix Modifier | fumed silica | 7 wt% |
| Skin Penetration Enhancer | laurydone | 2-4 wt% |
| | dimethyl sulfoxide | 0-5 wt% |

Where a patent, application, or publication contains express definitions, those definitions should be understood to apply to the cited patent, application or publication in which they are found and not to the present application unless otherwise indicated.

## Claims

1. A composition for preparing a patch for transdermal delivery of donepezil, comprising:
an adhesive matrix comprising
(a) 5-50 wt% donepezil free base;
(b) 5-50 wt% of at least one adhesive polymer;
(c) 5-40 wt% of at least one solubility enhancer that is an ester of a dicarboxylic acid; and
(d) 1-20 wt% of at least one lipophilic permeation enhancer that is selected from a fatty acid, an α-hydroxy acid, a β-hydroxy acid, and a keto carboxylic acid.

2. The composition of claim 1, wherein the fatty acid is an unsaturated fatty acid.

3. The composition of claim 1, wherein the fatty acid is selected from oleic acid, linoleic acid, and linolenic acid.

4. The composition of claim 1, wherein the keto carboxylic acid is levulinic acid.

5. The composition of claim 1, wherein the α-hydroxy acid is lactic acid or glycolic acid.

6. The composition of any one of claims 1 to 5, wherein the at least one solubility enhancer is selected from dimethyl succinate and diethyl succinate.

7. The composition of any one of claims 1 to 6, further comprising at least a second permeation enhancer.

8. The composition of claim 7, wherein the second permeation enhancer is selected from methyl laurate, propylene glycol monolaurate, glycerol monolaurate, glycerol monooleate, lauryl lactate, laurydone, and myristyl lactate.

9. The composition of any one of claims 1 to 8, wherein the polymer adhesive is a pressure sensitive polymer adhesive.

10. The composition of any one of claims 1 to 9, wherein the at least one adhesive polymer is selected from an acrylic polymer, an acrylate polymer, an acrylic ester polymer, polymers comprising a pyrrolidone group, polyisobutylenes, polybutenes, polymers comprising an acetate group, and co-polymers thereof.

11. The composition of claim 10, wherein the acrylate polymer is present in the adhesive matrix at an amount of 35-50 wt%.

12. The composition of claim 10, wherein the at least one adhesive polymer is selected from a cross-linked polyvinylpyrrolidone, a soluble polyvinylpyrrolidone, and co-polymers thereof.

13. The composition of claim 12, wherein the at least one adhesive polymer is a polyvinylpyrrolidone-vinyl acetate copolymer present in the adhesive matrix at an amount of 5-35 wt%.

14. The composition of claim 10, wherein the at least one adhesive polymer is a mixture of polyisobutylene and polybutene polymers where the mixture is present in the adhesive matrix at an amount of 35-50 wt%.

15. A composition for preparing a patch for transdermal delivery of donepezil, comprising:
an adhesive matrix comprising:
(a) 5-20 wt% donepezil in free base form;
(b) 2-6 wt% lauryl lactate or lauryl pyrrolidone carboxylate;
(c) 5-20 wt% dimethyl succinate;
(d) 0-15% dimethyl sulfoxide;
(e) 1-10 wt% levulinic acid or lactic acid;
(f) 3-20 wt% fumed silica and/or cross-linked polyvinyl pyrrolidone; and
(g) 35 to 80 wt% of an adhesive comprising a mixture of polyisobutylene and polybutene or a hydrogenated polybutene.

## Patentansprüche

1. Zusammensetzung zur Herstellung eines Pflasters zur transdermalen Verabreichung von Donepezil, umfassend:
Klebematrix, umfassend
(a) 5-50 Gew.□% Donepezil freie Base;
(b) 5-50 Gew.□% mindestens eines Klebpolymers
(c) 5-40 Gew.□% mindestens eines Löslichkeitsverbesserers, bei dem es sich um einen Ester von Dicarbonsäure handelt und
(d) 1-20 Gew.□% mindestens eines lipophilen Permeationsverstärkers, der aus einer Fettsäure, einer α-Hydroxysäure, einer β-Hydroxysäure und einer Ketocarbonsäure ausgewählt ist.

2. Zusammensetzung nach Anspruch 1, wobei es sich bei der Fettsäure um eine ungesättigte Fettsäure handelt.

3. Zusammensetzung nach Anspruch 1, wobei die Fettsäure aus Ölsäure, Linolsäure und Linolensäure ausgewählt ist.

4. Zusammensetzung nach Anspruch 1, wobei es sich bei der Ketocarbonsäure um Lävulinsäure handelt.

5. Zusammensetzung nach Anspruch 1, wobei es sich bei der Hydroxysäure um Milch- oder Glykolsäure handelt.

6. Zusammensetzung nach einem der Ansprüche 1 bis 5, wobei der mindestens eine Löslichkeitsverbesserer aus Dimethylsuccinat und Diethylsuccinat ausgewählt ist.

7. Verfahren nach einem der Ansprüche 1 bis 6, ferner umfassend mindestens einen zweiten Permeationsverstärker.

8. Zusammensetzung nach Anspruch 7, wobei der zweite Permeationsverstärker aus Methyllaurat, Propylenglykolmonolaurat, Glycerolmonolaurat, Glycerinmonooleat, Lauryllactat, Laururydon und Myristyllactat ausgewählt ist.

9. Zusammensetzung nach einem der Ansprüche 1 bis 8, wobei es sich bei dem Klebpolymer um ein druckempfindliches Klebpolymer handelt.

10. Zusammensetzung nach einem der Ansprüche 1 bis 9, wobei das mindestens eine Klebpolymer aus einem Acrylpolymer, einem Acrylatpolymer, einem Acrylesterpolymer, eine Pyrrolidongruppe umfassenden Polymeren, Polyisobutylenen, Polybutenen, eine Acetatgruppe umfassenden Polymeren und Copolymeren davon ausgewählt ist.

11. Zusammensetzung nach Anspruch 10, wobei das Acrylatpolymer in der Klebematrix in einer Menge von 35-50 Gew.□% vorliegt.

12. Zusammensetzung nach Anspruch 10, wobei das mindestens eine Klebpolymer aus einem vernetzten Polyvinylpyrrolidon, einem löslichen Polyvinylpyrrolidon und Copolymeren davon ausgewählt ist.

13. Zusammensetzung nach Anspruch 12, wobei es sich bei dem mindestens einen Klebpolymer um ein Polyvinylpyrrolidon-Vinylacetat-Copolymer handelt, das in der Klebematrix in einer Menge von 5-35 Gew.□% vorliegt.

14. Zusammensetzung nach Anspruch 10, wobei es sich bei dem mindestens einen Klebpolymer um eine Mischung aus Polyisobutylen- und Polybutenpolymeren handelt, wobei die Mischung in der Klebematrix in einer Menge von 35-50 Gew.□% vorliegt.

15. Zusammensetzung zur Herstellung eines Pflasters zur transdermalen Verabreichung von Donepezil, umfassend:
Klebematrix, umfassend
(a) 5-20 Gew.□% Donepezil in freier Basenform;
(b) 2-6 Gew.□% Lauryllactat oder Laurylpyrrolidoncarboxylat;
(c) 5-20 Gew.□% Dimethylsuccinat;
(d) 0-15 Gew.□% Dimethylsulfoxid;
(e) 1-10 Gew.□% Lävulinsäure oder Milchsäure;
(f) 3-20 Gew.□% pyrogenes Siliciumdioxid und/oder vernetztes Polyvinylpyrrolidon und
(g) 35 bis 80 Gew.□% eines eine Mischung aus Polyisobutylen und Polybuten oder ein hydriertes Polybuten umfassenden Klebstoffs.

## Revendications

1. Composition pour préparer un patch pour l'administration transdermique de donépézil, comprenant :
une matrice adhésive comprenant
(a) 5 à 50 % en poids de donépézil base libre ;
(b) 5 à 50 % en poids d'au moins un polymère adhésif ;
(c) 5 à 40 % en poids d'au moins un adjuvant de solubilité qui est un ester d'un acide dicarboxylique ; et
(d) 1 à 20 % en poids d'au moins un adjuvant de perméation lipophile qui est choisi parmi un acide gras, un α-hydroxyacide, un β-hydroxyacide, et un acide cétocarboxylique.

2. Composition selon la revendication 1, dans laquelle l'acide gras est un acide gras insaturé.

3. Composition selon la revendication 1, dans laquelle l'acide gras est choisi parmi l'acide oléique, l'acide linoléique et l'acide linolénique.

4. Composition selon la revendication 1, dans laquelle l'acide cétocarboxylique est l'acide lévulinique.

5. Composition selon la revendication 1, dans laquelle le α-hydroxyacide est l'acide lactique ou l'acide glycolique.

6. Composition selon l'une quelconque des revendications 1 à 5, dans laquelle l'au moins un adjuvant de solubilité est choisi parmi le succinate de diméthyle et le succinate de diéthyle.

7. Composition selon l'une quelconque des revendications 1 à 6, comprenant en outre au moins un deuxième adjuvant de perméation.

8. Composition selon la revendication 7, dans laquelle le deuxième adjuvant de perméation est choisi parmi les laurate de méthyle, monolaurate de propylène glycol, monolaurate de glycérol, monooléate de glycérol, lactate de lauryle, laurydone et lactate de myristyle.

9. Composition selon l'une quelconque des revendications 1 à 8, dans laquelle le polymère adhésif est un polymère adhésif autocollant.

10. Composition selon l'une quelconque des revendications 1 à 9, dans laquelle l'au moins un polymère adhésif est choisi parmi un polymère acrylique, un polymère d'acrylate, un polymère d'ester acrylique, des polymères comprenant un groupe pyrrolidone, des polyisobutylènes, des polybutènes, des polymères comprenant un groupe acétate, et des copolymères de ceux-ci.

11. Composition selon la revendication 10, dans laquelle le polymère d'acrylate est présent dans la matrice adhésive en une quantité de 35 à 50 % en poids.

12. Composition selon la revendication 10, dans laquelle l'au moins un polymère adhésif est choisi parmi une polyvinylpyrrolidone réticulée, une polyvinylpyrrolidone soluble, et des copolymères de celles-ci.

13. Composition selon la revendication 12, dans laquelle l'au moins un polymère adhésif est un copolymère de polyvinylpyrrolidone-acétate de vinyle présent dans la matrice adhésive en une quantité de 5 à 35 % en poids.

14. Composition selon la revendication 10, dans laquelle l'au moins un polymère adhésif est un mélange de polymères de polyisobutylène et de polybutène où le mélange est présent dans la matrice adhésive en une quantité de 35 à 50 % en poids.

15. Composition pour préparer un patch pour l'administration transdermique de donépézil, comprenant :
une matrice adhésive comprenant :
(a) 5 à 20 % en poids de donépézil sous forme de base libre ;
(b) 2 à 6 % en poids de lactate de lauryle ou pyrrolidonecarboxylate de lauryle ;
(c) 5 à 20 % en poids de succinate de diméthyle ;
(d) 0 à 15 % de diméthylsulfoxyde ;
(e) 1 à 10 % en poids d'acide lévulinique ou d'acide lactique ;
(f) 3 à 20 % en poids de silice pyrogénée et/ou polyvinylpyrrolidone réticulée ; et
(g) 35 à 80 % en poids d'un adhésif comprenant un mélange de polyisobutylène et de polybutène ou d'un polybutène hydrogéné.
